# EUROPEAN PATENT APPLICATION

(11) **EP 3 744 253 A1**
(43) Date of publication of application: **02.12.2020**
(21) Application number: 19176889.4
(22) Date of filing: 28.05.2019
(51) Int. Cl.: A61B 5/16, A61B 5/18, A61B 5/1172, A61B 5/1171, A61B 5/00, B60L 3/02

(54) **TESTING APPARATUS FOR TESTING THE CONDITION OF A USER OF A DEVICE, AND TRANSPORT DEVICE COMPRISING SUCH A TESTING APPARATUS**

(71) Applicant: Kubatronik Leiterplatten GmbH, 73312 Geislingen/Steige (DE)
(72) Inventor: Gal, Avraham, 7127906 LOD (IL)
(74) Representative: DREISS Patentanwälte PartG mbB

(57) **Abstract**

A testing apparatus (18) for testing the condition of a user of a device, in particular of a transport device (10), comprises a generator means (24) for generating a random sequence of n alphanumeric digits, output means (26) configured to display the generated sequence of n alphanumeric digits, input means (28) configured as a keyboard (42) to receive an input of alphanumeric digits by the user, and control and processing means (22) configured to allocate a first condition to the user if the user, within a test sequence, correctly inputs all n digits in inverse sequence displayed on the output means (26) within a predetermined time range after a start of the test sequence, and configured to allocate a second condition to the user if the user, within a test sequence, does not correctly input all n digits in inverse sequence displayed on the output means (26) within the predetermined time range after a start of the test sequence.

## Description

The present invention relates to a testing apparatus for testing the condition of a user of a device, and to a transport device comprising such a testing apparatus, according to the preambles of the independent claims.

EP 2 485 645 B1 discloses a testing apparatus and a method for testing sustained attention of a person using so-called "mini mental state examination". This type of examination is used in psychology to provide a screening procedure suitable for the detection of cognitive deficits. Mini mental state examination has become the most widely used instrument in the diagnosis of dementia and Alzheimer's disease.

It is also known in the art to provide devices for detecting the condition of a driver of a car and to allow the driver to start the engine of the car only if the detected condition is deemed to be appropriate for driving the car. Such devices may comprise a tube into which the user must blow, and analyzing means analyzing the air blown into the tube.

It is an object of the present invention to provide a testing apparatus for testing the mental and/or physical condition of a user of a device having an improved reliability, being easy to use and being cost efficient.

This object is achieved with a testing apparatus having the features of claim 1 and with a transport device having the features of the independent claim. Further embodiments are claimed in the dependent claims. Other features, which may be important for the invention, are given in the following description and in the attached drawing, wherein these features may be relevant for the invention alone or in various combinations as well.

The inventive testing apparatus may be used for testing the condition of a user of a device, in particular of a transport device. Such a transport device may be a car, a truck, a bus, a motorbike, a bicycle, an aircraft, a railway locomotive, a boat, or the like. However, the proposed testing apparatus also may be used for testing the condition of the operator of a machine, for example a production machine, or the like. The testing apparatus may be an apparatus separate from the device, or may be integrated into the device. It comprises a control and processing means having a generator means for generating a random sequence of n alphanumeric digits, n being an integer. The alphanumeric digits preferably comprise only Latin letters and/or Arabic numbers, but also may comprise digits of other languages/writings, and/or may comprise signs, such as question marks, or the like. The generator is configured to ensure that with a sufficiently high probability only such subsequent sequences are generated which are sufficiently different from each other. Normally, the generator may be realized by software, which is stored on a storage means and executed on a microprocessor.

The testing apparatus also comprises an output means, which is configured to display the generated sequence of n alphanumeric digits to the user. The output means may be any type of visual display, such as LCD, LED, TFT, OLED, or the like. It is preferred that a dark background color is used and that the digits are displayed on the display as dark characters in separate bright fields being located close to each other. Further, it is preferred that the digits, preferably the above mentioned fields, are displayed as a horizontal row, and that all digits are displayed at the same time.

Furthermore, the testing apparatus comprises an input means, which is configured as a keyboard to receive an input of alphanumeric digits by the user. The keyboard might be a means which is physically separate from the output means, for example a classical computer keyboard. Preferably, the input means is realized as a virtual keyboard on a touch sensitive screen, which preferable is the same screen as used for the output means. The keyboard preferably covers the entire Latin alphabet as well as all Arabic numbers from 0 to 9. It's layout may comply with national or regional traditions, that is, for example, being a QWERTZ layout in Germany, an AZERTY layout in France, a QWERTY layout in the US, and so on. If a virtual keyboard is used, the layout may be automatically defined depending on a language selected by the user.

The testing apparatus according to the invention may be used in a test sequence in order to test the condition of a user of a device. In the test sequence, the sequence or string of n alphanumeric digits is prompted to the user by means of the output means, and the user then must enter the sequence or string of n alphanumeric digits in inverse sequence within a predetermined time range by means of the input means. The user only passes the test if he or she enters all alphanumeric digits in the correct inverse sequence within the predetermined time range (or, of course, within a time shorter than the predetermined time range).

In order to allow a user to complete such a test sequence, according to the invention, the control and processing means is configured to allocate a first condition to the user if the user, within a test sequence, correctly inputs all n digits in inverse sequence shown on the display within a predetermined time range after a start of the test sequence. This first condition means that, according to the test, the user has sufficient mental and physical capacity to use the device, in particular the transport device.

Further, the control and processing means is configured to allocate a second condition to the user if the user, within a test sequence, does not correctly input all n digits in inverse sequence shown on the display within the predetermined time range after a start of the test sequence. This second condition means that, according to the test, the user does not have sufficient mental and physical capacity to use the device. Such a condition, for example, may be given in the case that the user is drunk and/or has taken drugs.

Thus, a short diagnostic test will diagnose a user's fitness or incapacity for using the device. If the user is not fit to use the device, the testing apparatus according to the invention will directly detect this condition, and may, for example, prevent the device from being used or operated by the user. By consequence, the testing apparatus according to the invention greatly enhances safety when a user wants to use or operate a device.

The invention addresses the needs of insurance companies, large vehicle fleet operators, airlines (pilots might need to undergo the test on very long flights at specific moments of time), licensing authorities, but also private persons. It allows an easy and fast diagnosis and does not require any special skills beyond reading comprehension and answering. Installation is possible at a very low cost, since the inventive apparatus can be realized by using software and common low cost components.

In a further preferred embodiment of the invention, n (number of digits prompted to the user) is 7 to 10, more preferably 8. Clinical trials have shown that a number of 7 to 10, more preferably 8 digits, which are prompted by the output means and which then have to be entered by the user via the input means in reverse order, is sufficient in order to reliably assess the condition of the user, and limits the duration of the test sequence to a reasonable amount.

In a further preferred embodiment of the invention, the time range for the test sequence is approx. 2-3 sec./digit, more preferably approx. 2.5 sec./digit. This means that, when a sequence or string of 8 digits is prompted, the user must enter the sequence in reverse order within a time range of 20 seconds in order to pass the test. Again, clinical trials have shown that the above-mentioned values provide particularly reliable test results.

In a further preferred embodiment of the invention, the testing apparatus comprises an interface to a control device of the machine, wherein the control and processing means provides a signal to the interface, the signal indicating whether the first or the second condition has been allocated to the user. This allows to automatically lock the device, which the user wants to use or operate, in case that the test sequence resulted in the second condition being allocated to the user. Thus, safety is enhanced because the user is automatically prevented from using or operating the device if he is found to be in the second condition.

In a further preferred embodiment of the invention, the control and processing means is configured to allow, after the second condition was allocated to the user, only a predetermined number of retries. By way of example, the total number of test sequences which are allowed to be executed immediately one after the other may be limited to three. Thus, any abuse of the testing apparatus is prevented.

In a further preferred embodiment of the invention, the control and processing means is configured to allow, after the second condition was allocated to the user, a retry of a test sequence or of a limited number of test sequences with a new random sequence of n alphanumeric digits only after a predetermined time delay. This takes into account that a user of a device may suffer from a temporary incapacity. It is particularly preferred that the control and processing means allows the user to execute the test sequence up to three consecutive times and, if also the third test sequence is failed, then locks the option for additional attempts for a time delay of 15 minutes. Therefore, if the temporary incapacity after a certain time has passed, the user may try again to undergo the test sequence, and the user will be able to operate the device in case that he or she then passes the test. Furthermore, the time delay may increase from retry to a subsequent retry or from a number of retries to a subsequent number of retries, respectively.

In a further preferred embodiment of the invention, the testing apparatus comprises identification means for identifying the person of the user inputting digits at the input means. With such an identification means the first condition may only be allocated to a user if the person using the testing apparatus has been identified by the identification means as being authorized to use the testing apparatus and as being authorized to operate the device. Thus, it is prevented that a second person, which is not the person operating the device or which is a person not authorized to operate the device, undergoes the test sequence and passes the test in place of the actual person intending to operate the device. This makes it more difficult to swindle.

In a further preferred embodiment of the invention, the identification means comprises a fingerprint sensor and/or a face recognition means. These types of identification means are reliable and low cost.

In a further preferred embodiment of the invention, the testing apparatus comprises analyzing means analyzing at least one parameter characterizing the user input in case that the second condition has been allocated to the user. This allows not only making a "digital" yes/no decision, but rather to identify the specific condition of the user making him or her incapable to operate the device.

In a further preferred embodiment of the invention, the parameter is at least one parameter of the following group: location in the sequence of a wrong input; point in time of a wrong input within the time range; number of wrong inputs; number of inputs within the time range; relation between the number of correct inputs and the number of wrong inputs; type of wrong input. It was found out that these parameters provide for a reliable identification of the specific condition of the user.

In a further preferred embodiment of the invention, the analyzing means allocates a medical symptom depending on the analyzing result. The medical symptom may be at least one medical symptom of the following group: vertigo and/or dizziness; vision field problem; response time problem; dementia and/or Alzheimer; alcohol blurring; emotional insensitivity; weak reflex; coordination problem; short memory problem; cognition problem; limitations in shoulder movements, hands and/or fingers. The inventive testing apparatus therefore allows to identify at least 12 symptoms and therefore to provide a differentiated diagnosis of the reason for the incapacity of the user.

The invention also relates to a transport device, such as a vehicle or an aircraft, comprising control means for controlling use of the transport device.

In order to enhance safety when operating the transport device, the transport device comprises an interface configured to receive a signal from a testing apparatus as described above, the signal indicating whether the first or the second condition has been allocated to the user, wherein the control means is configured to only allow operation of the transport device in case that the signal indicates that the first condition has been allocated to the user. The interface may be a wired interface or a wireless interface, such as Bluetooth or WiFi. In the latter case, the testing apparatus may be realized for example by a smartphone or a tablet computer, and the testing sequence may be realized by program code executed on the smart phone or tablet computer. The invention considerably enhances the operational safety of the transport device.

In a further preferred embodiment of the transport device , a testing apparatus as described above is integrated into the transport device. This makes it more difficult to manipulate the testing apparatus.

In a further preferred embodiment of the transport device, a touch sensitive display of the transport device comprises the output means and the input means of the testing apparatus as described above. Such touch sensitive displays are very common in today's cars and aircrafts such that no additional means is needed.
- Figure 1: is a schematic representation of a transport device having a testing apparatus for testing the condition of a user or operator of the transport device; and
- Figures 2-11: are plain views on a combined output and input means of the testing apparatus of figure 1 at different times steps during a test sequence.

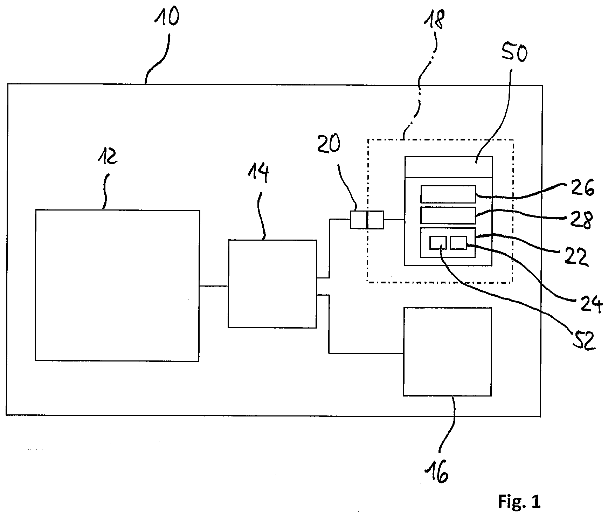

A transport device in figure 1 is generally denoted with reference numeral 10. It comprises an engine 12, a controller 14 and a start button 16 for starting the engine 12 by a user. The transport device 10 may be a car, a truck, a bus, a motorbike, a bicycle, an aircraft, a boat, a railway locomotive, or the like. The engine 12 may be a combustion engine or an electric engine. The controller 14 may be a single unit or may comprise a number of sub-controllers (not shown). The controller 14 normally comprises a microprocessor and at least one storage with program code stored thereon.

The transport device 10 also comprises a testing apparatus 18, which is shown as a dot and dash lined box. The testing apparatus 18 communicates with the controller 14 of the transport device 10 through an interface 20. The interface 20 may be realized as a hard wired device, but it also may be realized as a wireless device, such as Bluetooth or WiFi. The testing apparatus 18 may be an apparatus separate from the transport device 10, or may be integrated into the transport device 10.

It comprises a control and processing means 22 having a generator means 24 for generating a random sequence of n alphanumeric digits, n being an integer. The control and processing means 22 normally comprises a microprocessor and at least one storage with program code stored thereon. The generator means 24 may be a random number generator and normally is realized by software, which is stored on a storage means and executed by a microprocessor.

The person skilled in the art readily understands how to realize such a generator means 24. For example, it is known to realize non-deterministic random number generators or deterministic random number generators. The generator means 24 is configured to ensure that with a sufficiently high probability only such subsequent sequences are generated which are sufficiently different from each other.

The testing apparatus 18 also comprises an output means 26, which is configured to display the generated sequence of n alphanumeric digits to the user of the transport device 10. The output means 26 may be any type of visual display, such as LCD, LED, TFT, OLED, or the like. As will be shown hereinafter, it is preferred that the digits, preferably the above mentioned fields, are displayed as a horizontal row, and that all digits are displayed at the same time.

The testing apparatus 18 also comprises an input means 28, which is configured as a keyboard to receive an input of alphanumeric digits by the user of the transport device 10. The keyboard might be a means which is physically separate from the output means 26, for example a classical computer keyboard. Preferably, the input means 28 is realized as a virtual keyboard on a touch sensitive screen, which preferable is the same screen as used for the output means 26. For example, the output means 26 and the input means 28 may be realized by a multifunctional display in the cockpit of the transport device 10.

The alphanumeric digits preferably comprise only Latin letters and/or Arabic numbers, but also may comprise digits of other languages/writings, and/or may comprise signs, such as question marks, or the like. The keyboard preferably covers the entire Latin alphabet as well as all Arabic numbers from 0 to 9. It's layout may comply with national or regional traditions, that is, for example, being a QWERTZ layout in Germany, an AZERTY layout in France, a QWERTY layout in the US, and so on. If a virtual keyboard is used, the layout may be automatically defined depending on a language selected by the user.

The testing apparatus 18 may be used in a test sequence in order to test the condition of a user of the transport device 10. In the test sequence, a sequence or string of n alphanumeric digits is prompted by means of the output means 26 to the user, and the user then must enter the sequence or string of n alphanumeric digits in inverse sequence within a predetermined time range by means of the input means 28. The user only passes the test if he or she enters all alphanumeric digits in the correct inverse sequence within the predetermined time range (or, of course, within a time shorter than the predetermined time range).

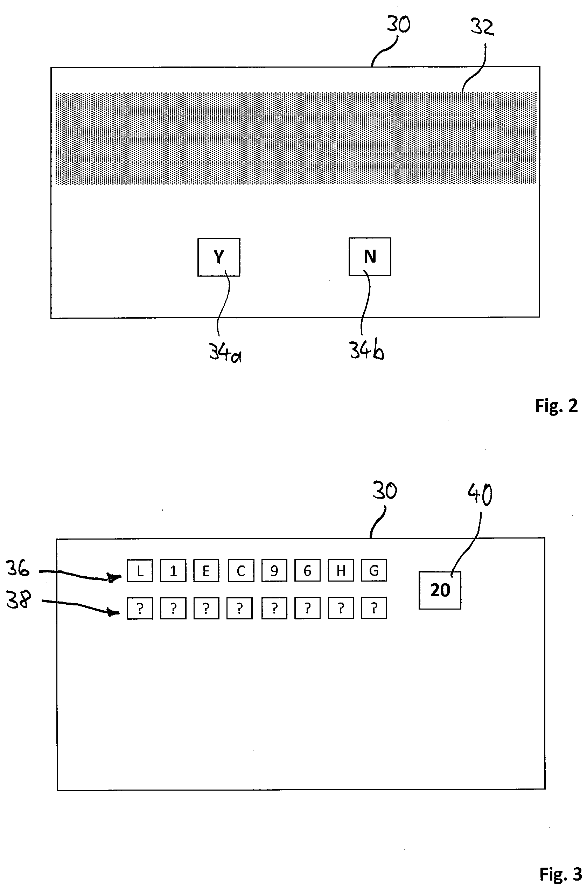

The possible courses of such a test sequence will now be explained in more detail with reference to figures 2-11. Figures 2-11 show a plain view on a combined output means 26 and input means 28 in the form of a touch sensitive display 30. As can be seen from figure 2, when starting the test sequence, the upper part of the touch sensitive display 30 is used to prompt text or graphic information to the user. This text or graphic information is indicated in figure 2 by a grey area 32.

As an example, the text or graphic information 32 may provide instructions to the user as to how to use the testing apparatus 18 and how to undergo the test sequence. More specifically, the text or graphic information 32 may instruct the user: "please type the displayed string on backward", and then a generic example may be given. Furthermore, the text or graphic information 32 may indicate to the user the time range within which the test sequence must be completed. For example, in the present case, the time range may be 20 seconds. Also, the text or graphic information 32 may comprise a "ready" question, such as: "are you ready?", and may comprise two buttons or icons 34a and 34b, one designated with "yes" and one designated with "no". Alternatively, the icons 34a and 34b might be designated with "start" and "cancel", respectively. The person skilled in the art readily knows that any other text or graphic information 32 may be prompted to the user which helps him or her to understand the course of the test sequence and to start the test sequence.

As can be seen from figure 3, when the test sequence has been started by touching the icon 34a "yes", two horizontal rows 36 and 38 of fields and, on the right side of these rows 36 and 38, a larger field 40 appear. The upper row 36 consists in the present exemplary case of eight almost quadratic fields, which are arranged close to each other, with a small gap between adjacent fields. However, in other embodiments, another number of fields may be used, the number ranging from 7 to 10. Each field of the upper row 36 comprises one alphanumeric digit. The sequence of the alphanumeric digits from left to right has been generated by the random generator means 24. By consequence, the upper row 36 can be considered as the above mentioned output means 26 which is configured to display the generated sequence of eight alphanumeric digits. By way of example, the sequence prompted by the upper row 36 in figure 3 is:
L 1 E C 9 6 H G

The lower row 38 equally consists of eight fields, which are identically sized to the fields of the upper row 36. Each field of the lower row 38 is directly below and close to a field of the upper row 36, with a small gap between the fields of the lower row 38 and the upper row 36. Each field of the lower row 38 comprises, at this moment of time during the test sequence, a question mark. Alternatively, the fields of the lower row 38 might simply be blank.

In the larger field 40, a number is shown. As will be shown below, the field 40 is a counter which indicates to the user of the testing apparatus 18 during the test sequence the time left until the end of the test sequence. By way of example, field 40 indicates, at the very beginning of the test sequence, that the user has 20 seconds to complete the test sequence.

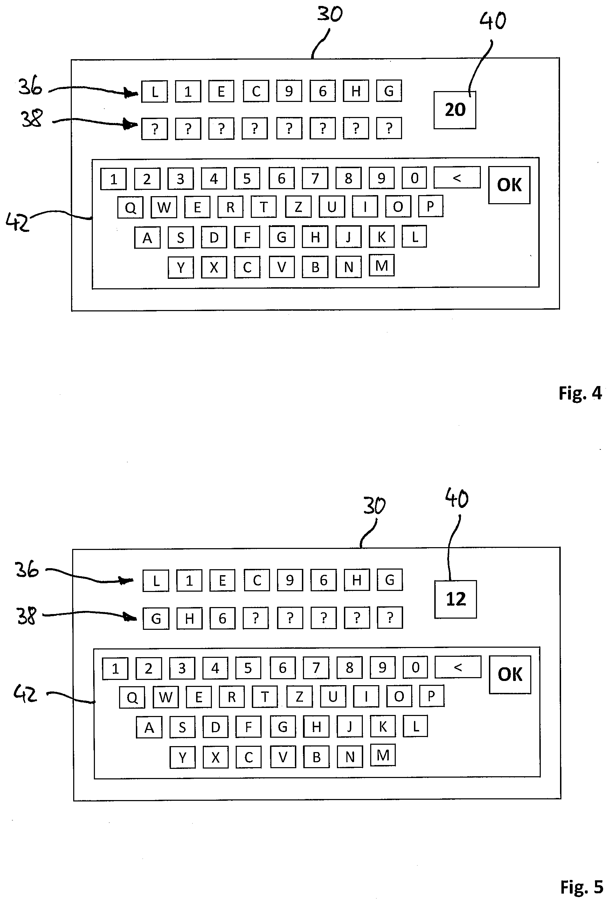

Immediately after the screen content of figure 3 has been displayed, in the lower part of the touch sensitive display 30 a virtual keyboard 42 is displayed, as can be seen from figure 4. In the present case, the virtual keyboard 42 has a QWERTZ layout as it is used in Germany. In other countries or regions, other layouts of keyboards 42 may be displayed, depending on the national or regional traditions. The keyboard 42 also comprises an "okay" icon and an "undo" icon. The keyboard 42 thus may be considered as the input means 28 mentioned above. With the appearance of the keyboard 42, the time counter 40 starts to count the time left, that is from 20 to 0.

The user now must type with his finger on the virtual keyboard 42 the string of digits or characters indicated in the upper row 36, however in the inverse sequence. The virtual keyboard 42 is sized such that the user only can use one single finger to type in the characters. This means in the present exemplary test sequence that the user, in order to pass the test, must type within a maximum time range of 20 seconds the following string:
G H 6 9 C E 1 L

The actual inputs of the user by means of the virtual keyboard 42 are prompted in the fields of the lower row 38, wherein the inputs at the virtual keyboard 42 fill the lower row 38 from left to right. By way of example, figure 5 shows a possible situation at a moment of time when there are still 12 seconds left until the end of the test sequence. As can be seen from figure 5, the user has correctly input the first three digits.

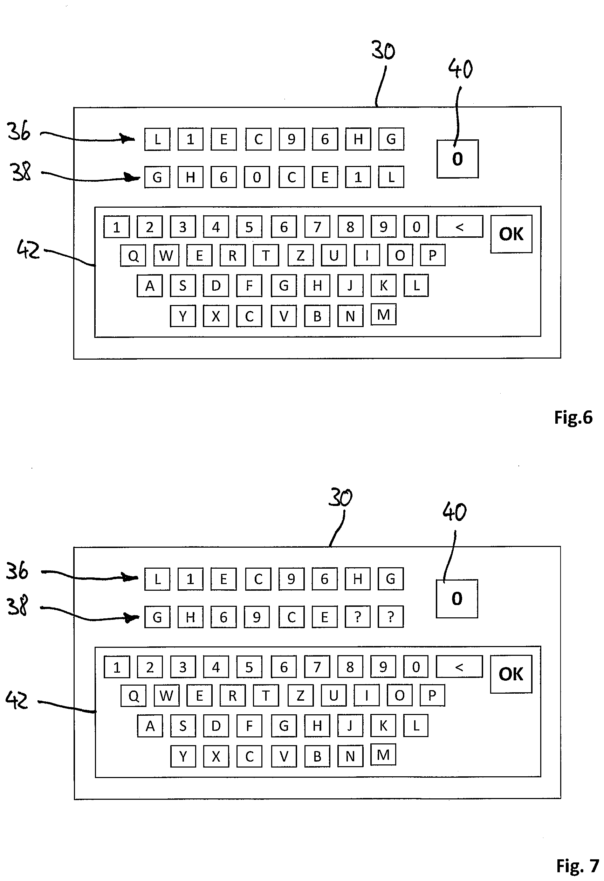

Figure 6 shows a situation, where the user has completed the input of all eight digits prior to the end of the time range of 20 seconds. However, by way of example, it is shown in figure 6 that the user did not correctly input the fourth digit, since he or she typed a "0" instead of a "9". This means that the user did not correctly input all eight digits in inverse sequence displayed on the output means 26 within the time range of 20 seconds.

Figure 7 shows a similar situation. Other than in figure 6, while all inputs of the user are correct, the user was not able to complete the input of all eight digits prior to the end of the time range of 20 seconds. As can be seen from figure 7, the last two fields of the lower row 38 still indicate the question mark. This also means that the user did not correctly input all eight digits in inverse sequence displayed on the output means 26 within the time range of 20 seconds.

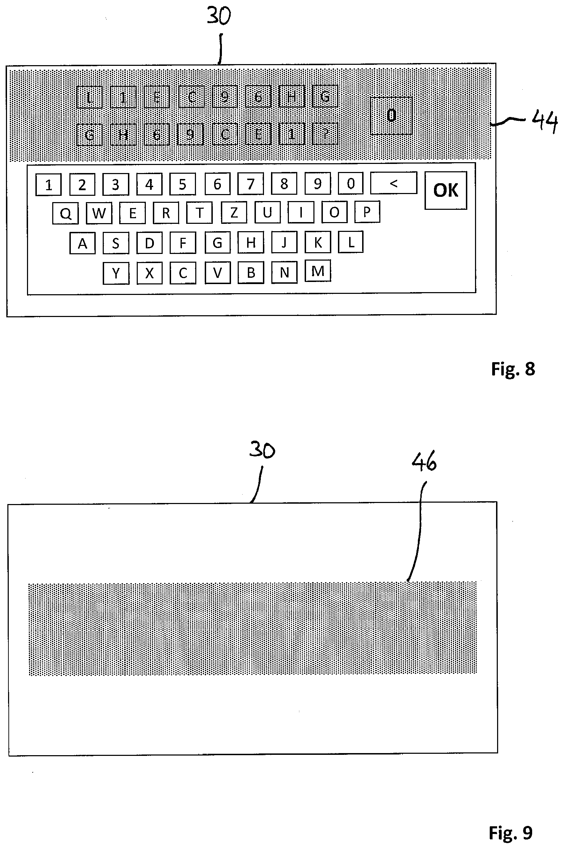

By consequence, since the test was failed, the control and processing means 22 allocates a second condition to the user. This second condition means that, according to the testing apparatus 18, the user is not in a condition to safely operate the transport device 10. When the control and processing means 22 allocates the second condition to the user, it sends a corresponding signal through the interface 22 to the controller 14 of the transport device 10. This signal causes that, even when the user pushes the start button 16 of the transport device 10, the engine 12 does not start, such that the user is unable to operate the transport device 10. Also, the touch sensitive display 30 indicates graphically or textually to the user that the test was failed. This is shown in figure 8 by a grey area 44, which on the touch sensitive display 30 may be a striking color, such as red.

As shown in figure 9, thereafter, the touch sensitive display 30 may prompt to the user another textual or graphical information 46, which for example may comprise the question, whether the user wants to retry the test sequence. In this context, it may be mentioned that the control and processing means 22 may be configured to allow, after the second condition was allocated to the user, only a predetermined number of retries within a certain time range. By way of example, the total number of test sequences which are allowed to be executed immediately one after the other may be limited to three.

Furthermore, the control and processing means 22 may be configured to allow, after the second condition was allocated to the user, a retry of a test sequence or of a limited number of test sequences with a new random sequence of n alphanumeric digits only after a predetermined time delay. By way of a specific example, the control and processing means 22 may allow the user to execute the test sequence up to three consecutive times. If also the third test sequence was failed, the control and processing means 22 locks the option for additional attempts for a certain time delay, for example for 15 minutes. The time delay may increase from retry to a subsequent retry or from a number of retries to a subsequent number of retries, respectively. For example, the first number of retries may be allowed after a time delay of 15 minutes, and a second number of retries then may be allowed only after a time delay of 1 hour.

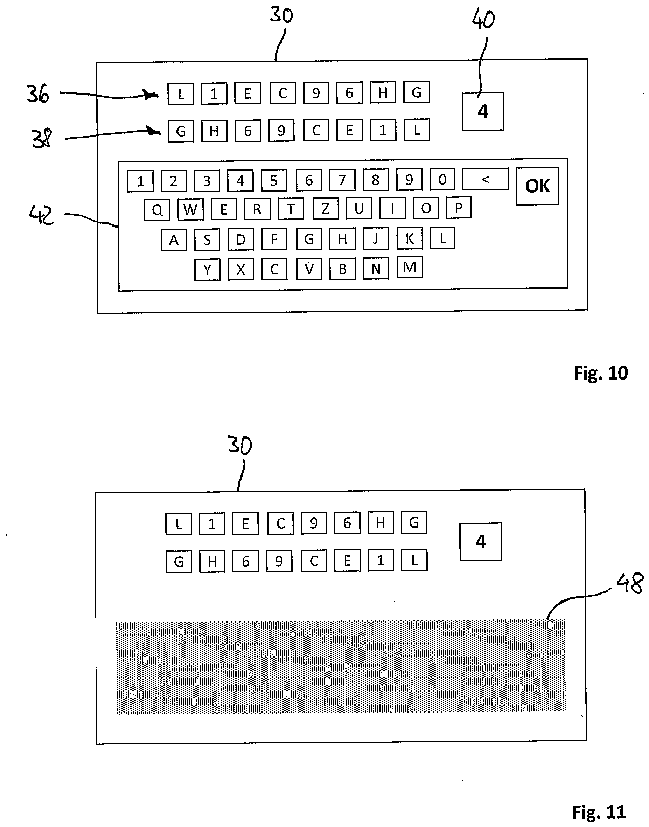

Figure 10 shows a situation at the end of a test sequence where the user has correctly input all eight digits in the inverse sequence prior to the end of the time range of 20 seconds. This means that the control and processing means 22 allocates a first condition to the user, this first condition indicating that the user is in a condition to safely operate the transport device 10. The user now may press the "okay" icon or simply wait until the time of 20 seconds has lapsed. As shown in figure 11, the touch sensitive display 30 then will prompt an information 48 to the user that the test was passed and that he may now start the engine by pressing the start button 16. Accordingly, the control and processing means 22 sends a corresponding signal through the interface 22 to the controller 14 of the transport device 10 allowing start of the engine 12.

As is shown in figure 1, the testing apparatus 18 also may comprise identification means 50 for identifying the person of the user who is inputting digits at the input means 28. The identification means 50 may comprise a fingerprint sensor and/or a face recognition means.

Furthermore, as is shown in figure 1, the testing apparatus 18 also may comprise analyzing means 52 for analyzing at least one parameter characterizing the user input in case that the second condition has been allocated to the user. The parameter is at least one parameter of the following group: location in the sequence of a wrong input; point in time of a wrong input within the time range; number of wrong inputs; number of inputs within the time range; relation between the number of correct inputs and the number of wrong inputs; type of wrong input.

The analyzing means 52 allocates a medical symptom depending on the analyzing result. The medical symptom is at least one medical symptom of the following group: vertigo and/or dizziness; vision field problem; response time problem; dementia and/or Alzheimer; alcohol blurring; emotional insensitivity; weak reflex; coordination problem; short memory problem; cognition problem; limitations in shoulder movements, hands and/or fingers.

## Claims

1. A testing apparatus (18) for testing the condition of a user of a device, in particular of a transport device (10), comprising an output means (26) for providing an output to the user, an input means (28) for receiving a user input, a control and processing means (22) for controlling the output means (26) and the input means (28) and for processing the user input, **characterized in that**
the control and processing means (22) comprises a generator means (24) for generating a random sequence of n alphanumeric digits,
the output means (26) is configured to display the generated sequence of n alphanumeric digits,
the input means (28) is configured as a keyboard (42) to receive an input of alphanumeric digits by the user,
the control and processing means (22) is configured to allocate a first condition to the user if the user, within a test sequence, correctly inputs all n digits in inverse sequence displayed on the output means (26) within a predetermined time range after a start of the test sequence, and is configured to allocate a second condition to the user if the user, within a test sequence, does not correctly input all n digits in inverse sequence displayed on the output means (26) within the predetermined time range after a start of the test sequence.

2. The testing apparatus (18) according to claim 1, wherein n is 7 to 10, more preferably 8.

3. The testing apparatus (18) according to at least one of claims 1-2, wherein the time range is approx. 2-3 sec./digit, more preferably approx. 2.5 sec./digit.

4. The testing apparatus (18) according to at least one of the preceding claims, wherein it comprises an interface (20) to a control device of the machine, and wherein the control and processing means (22) is configured to provide a signal to the interface (20), the signal indicating whether the first or the second condition has been allocated to the user.

5. The testing apparatus (18) according to at least one of the preceding claims, wherein the control and processing means (22) is configured to allow, after the second condition was allocated to the user, only a predetermined number of retries.

6. The testing apparatus (18) according to claim 5, wherein the control and processing means (22) is configured to allow, after the second condition was allocated to the user, a retry of a test sequence or a retry of a predetermined number of test sequences with new random sequences of n alphanumeric digits only after a predetermined time delay.

7. The testing apparatus (18) according to at least one of the preceding claims, wherein it comprises identification means (50) for identifying the person of the user inputting digits at the input means (28).

8. The testing apparatus (18) according to claim 7, wherein the identification means (50) comprises a fingerprint sensor and/or a face recognition means.

9. The testing apparatus (18) according to at least one of the preceding claims, wherein it comprises analyzing means (52) configured to analyze at least one parameter characterizing the user input in case that the second condition has been allocated to the user.

10. The testing apparatus (18) according to claim 9, wherein the parameter is at least one parameter of the following group: location in the sequence of a wrong input; point in time of a wrong input within the time range; number of wrong inputs; number of inputs within the time range; relation between the number of correct inputs and the number of wrong inputs; type of wrong input.

11. The testing apparatus (18) according to at least one of claims 9 and 10, wherein the analyzing means (52) allocates a medical symptom depending on the analyzing result.

12. The testing apparatus according (18) to claim 11, wherein the medical symptom is at least one medical symptom of the following group: vertigo and/or dizziness; vision field problem; response time problem; dementia and/or Alzheimer; alcohol blurring; emotional insensitivity; weak reflex; coordination problem; short memory problem; cognition problem; limitations in shoulder movements, hands and/or fingers.

13. A transport device (10), such as vehicle or aircraft, comprising control means (14) for controlling use of the transport device (10), **characterized in that** it comprises an interface (20) configured to receive a signal from a testing apparatus (18) according to at least one of the preceding claims, the signal indicating whether the first or the second condition has been allocated to the user, wherein the control means (14) is configured to only allow operation of the transport device (10) in case that the signal indicates that the first condition has been allocated to the user.

14. The transport device (10) of claim 13, wherein a testing apparatus (18) according to at least one of claims 1-12 is integrated into the transport device (10) .

15. The transport device (10) of claim 14, wherein a touch sensitive display (30) of the transport device (10) comprises the output means (26) and the input means (28) of the testing apparatus (18) according to at least one of claims 1-12.
